Europäisches Patentamt

(19) European Patent Office

Office européen des brevets

(11) EP 1 239 917 B1

(12) EUROPEAN PATENT SPECIFICATION

(45) Date of publication and mention
of the grant of the patent:
**11.05.2005 Bulletin 2005/19**

(51) Int Cl.⁷: **A61M 37/00**, A61N 1/30,
A61H 1/00, A61H 39/00,
A61B 5/00, A61B 17/20

(21) Application number: **00984209.7**

(22) Date of filing: **07.12.2000**

(86) International application number:
**PCT/US2000/033582**

(87) International publication number:
**WO 2001/041864 (14.06.2001 Gazette 2001/24)**

(54) **SKIN TREATMENT APPARATUS FOR SUSTAINED TRANSDERMAL DRUG DELIVERY**

HAUTBEHANDLUNGSVORRICHTUNG ZUR VERLÄNGERTEN TRANSDERMALEN
VERABREICHUNG VON MEDIKAMENTEN

DISPOSITIF DE TRAITEMENT CUTANE POUR ADMINISTRATION TRANSDERMIQUE
PROLONGEE D'UN MEDICAMENT

(84) Designated Contracting States:
**AT BE CH CY DE DK ES FI FR GB GR IE IT LI LU
MC NL PT SE TR**

(30) Priority: **10.12.1999  US 172704 P**

(43) Date of publication of application:
**18.09.2002  Bulletin 2002/38**

(73) Proprietor: **ALZA Corporation
Mountain View, CA 94039-7210 (US)**

(72) Inventors:
 • **CORMIER, Michel, J., N.
 Mountain View, CA 94043 (US)**
 • **TRAUTMAN, Joseph, C.
 Sunnyvale, CA 94087 (US)**
 • **KIM, Hyunok, L.
 Chino Hills, CA 91709 (US)**
 • **SAMIEE, Ahmad P.
 Fremont, CA 94555 (US)**
 • **NEUKERMANS, Armand, P.
 Portola Valley, CA 94028 (US)**
 • **EDWARDS, Bruce P.
 Palo Alto, CA 94304 (US)**
 • **LIM, Wai-Loong
 Menlo Park, CA 94025 (US)**
 • **POUTIATINE, Andrew, I.
 Menlo Park, CA 94025 (US)**

(74) Representative: **Williams, Paul Edwin et al
Ablett & Stebbing
Caparo House
101-103 Baker Street
London W1U 6FQ (GB)**

(56) References cited:
 WO-A-00/74763     WO-A-99/29365
 US-A- 4 114 619     US-A- 5 199 952
 US-A- 5 486 196     US-A- 5 857 983

**Description**

**TECHNICAL FIELD**

The present invention relates to transdermal agent delivery. More particularly, this invention relates to the transdermal delivery of macromolecular agents such as polypeptides, proteins, oligonucleotides and polysaccharides. The present invention relates to devices which have microprotrusions to pierce the outermost layer of a body surface (e.g., the skin) to enhance the transdermal flux of the agents during transdermal delivery.

**BACKGROUND ART**

Interest in the percutaneous or transdermal delivery of peptides and proteins to the human body continues to grow with the increasing number of medically useful peptides and proteins becoming available in large quantities and pure form. The transdermal delivery of peptides and proteins still faces significant problems. In many instances, the rate of delivery or flux of polypeptides through the skin is insufficient to produce a desired therapeutic effect due to the low transdermal permeability coefficient of macromolecules and the binding of the polypeptides to the skin. In addition, polypeptides and proteins are easily degraded during and after penetration into the skin, prior to reaching target cells. Likewise, the passive transdermal flux of many low molecular weight compounds is too limited to be therapeutically effective.

One method of increasing the transdermal delivery of agents relies on the application of an electric current across the body surface referred to as "electrotransport." "Electrotransport" refers generally to the passage of a beneficial agent, e.g., a drug or drug precursor, through a body surface, such as skin, mucous membranes, nails, and the like. The transport of the agent is induced or enhanced by the application of an electrical potential, which results in the application of electric current, which delivers or enhances delivery of the agent. The electrotransport of agents through a body surface may be attained in various manners. One widely used electrotransport process. iontophoresis involves the electrically induced transport of charged ions. Electroosmosis, another type of electrotransport process, involves the movement of a solvent with the agent through a membrane under the influence of an electric field. Electroporation, still another type of electrotransport, involves the passage of an agent through pores formed by applying a high voltage electrical pulse to a membrane. In many instances, more than one of these processes may be occurring simultaneously to a different extent. Accordingly, the term "electrotransport" is given herein its broadest possible interpretation, to include the electrically induced or enhanced transport of at least one charged or uncharged agent, or mixtures thereof, regardless of the specific mechanism or mechanisms by which the agent is actually being transported. Electrotransport delivery generally increases agent delivery and reduces polypeptide degradation during transdermal delivery.

Another method of increasing the agent flux involves pre-treating the skin with, or co-delivering with the beneficial agent, a skin permeation enhancer. A permeation enhancer substance, when applied to a body surface through which the agent is delivered, enhances its flux therethrough such as by reducing the electrical resistance of the body surface to the passage of the agent (i.e., during transdermal electrotrasport delivery), increasing the permselectivity and/or permeability of the body surface, creating hydrophilic pathways through the body surface, and/or reducing the degradation of the agent.

There also have been many attempts to mechanically penetrate or disrupt the skin in order to enhance the transdermal flux, such as, U.S. Patent Nos. 3,814,097 issued to Ganderton, et al., 5,279,544 issued to Gross, et al., 5,250,023 issued to Lee, et al., 3,964,482 issued to Gerstel, et al., Reissue 25,637 issued to Kravitz, et al., and PCT Publication Nos. WO 96/37155, WO 96/37256, WO 96/17648, WO 97/03718, WO 98/11937, WO 98/00193, WO 97/48440, WO 97/48441, and WO 97/48442. These devices use piercing elements of various shapes and sizes to pierce the outermost layer (i.e., the stratum corneum) of the skin. The piercing elements disclosed in these references generally extend perpendicularly from a thin, flat member, such as a pad or sheet. The piercing elements in some of these devices are extemely small, some having dimensions (i.e., a microblade length and width) of only about 25 - 400 μm and a microblade thickness of only about 5 - 50 μm. These tiny stratum corneum piercing/cutting elements are meant to make correspondingly small microslits/microcuts in the stratum corneum for enhanced transdermal agent delivery therethrough. In many instances, the microslits/microcuts in the stratum corneum have a length of less than 150 μm and a width which is substantially smaller than their length.

US 4,114,619 forming the basis of the preamble of claim 1 discloses an automatic injecting apparatus which comprises a hypodermic needle, a rim for snugly contacting a body surface, and a suction chamber which can pull the skin of the body surface into contact with the needle tip within the rim.

WO 99/29365A discloses a device for enhancing transdermal agent flux which comprises a sheet member having a plurality of microprotrusions for penetrating the skin, and a substantially incompressible agent reservoir housing contacting and extending across the sheet member.

## DESCRIPTION OF THE INVENTION

It has now been discovered that in the case of human skin, the small microslits/microcuts are quickly closed and sealed by the skin's natural healing processes. In some instances, the enhancement in transdermal agent flux provided by the microslits is completely eliminated within several hours of making the microslits. Thus, there is a need for a method and apparatus which can prevent, or at least delay the skin's natural healing processes in order to allow enhanced transdermal delivery of agents over longer periods of time (e.g., longer than about 1 hour).

The present invention provides enhanced and sustainable agent flux by applying tension to skin at the application site to maintain and/or enlarge pathways created by tiny skin penetrating elements. This enhanced agent flux is provided by applying a tension in the range of about 0.01 M Pa to about 10 M Pa, preferably about 0.05 M Pa to about 2 M Pa, to the skin after penetration of the body surface with the skin penetrating elements and holding the stretched condition during delivery of the agent. Applying tension to the body surface with the device of the present invention maintains and/or enlarges the pathways made in the outermost layer of the body surface. The tension also delays the closing and sealing of the pathways caused by the skin's natural healing process. The terms "applying tension" and "stretching" are used synonymously herein when referring to the pierced body surface.

In one embodiment of the present invention, a skin perforating apparatus is provided which can penetrate the outermost layer of the skin, i.e., stratum corneum, with a plurality of microprotrusions to form pathways in the form of microslits or microcuts through which an agent such as a drug can be introduced, i.e., delivered. The term "microprotrusions" as used herein refers to very tiny skin piercing elements, typically having a length of less than $500\mu$m, a width of less than $400\mu$m and a thickness of 5 to $100\mu$m, which make a correspondingly sized microcut or microslit in the skin.

The apparatus then uses stretching elements which engage the surface of the skin, such as with adhesive, and create opposing forces across the surface of the skin so as to create tension at the skin surface between the skin stretching elements. With the skin held in tension, the pathways through the skin are held open during treatment to facilitate sustainable and increased flux. The stretched skin provides improved flux particularly if electrotransport is used for sustained agent delivery through the skin.

In another embodiment of the invention, the apparatus comprises an expandable device with skin engaging portions which in use stretches the patient's skin and a relatively thin, flexible sheet, which in use is adapted to be placed in substantially parallel relation with the body surface to be pierced. The sheet has a plurality of microprotrusions extending from a body proximal side of the sheet for forming the pathways, i.e., microcuts/microslits in the outermost layer of the body surface. Preferably, the microprotrusions have a blade-like configuration and the expandable device is oriented to stretch the skin in a direction that is transverse to the plane of the microprotrusions.

The apparatus of the present invention can be used in connection with agent delivery, and in particular, transdermal drug delivery. Delivery devices for use with the present invention include, but are not limited to, electrotransport devices, passive devices, osmotic devices, and pressure-driven devices.

## BRIEF DESCRIPTION OF THE DRAWINGS

Many objects and advantages of the present invention will be apparent to those skilled in the art when this specification is read in conjunction with the attached drawings, wherein like reference numerals are applied to like elements, and wherein:

Figure 1 is a top plan view of a skin stretching device of the present invention applied to skin having a plurality of microslits therein;

Figure 2 is a cross-sectional view of the device shown in Figure 1 taken along line 2-2 of Figure 1;

Figure 3 is a diagrammatic top view of the pathways made in a body surface while being stretched using apparatus of the present invention;

Figure 4 is a perspective view of a first embodiment of a body surface stretching device in accordance with the present invention;

Figure 5 is a cross-sectional view of the device shown in Figure 4, taken along line 5-5 of Figure 4;

Figure 6 is an enlarged perspective view of a portion of the device shown in Figures 4 and 5;

Figures 7-9 illustrate the operation of a second embodiment of a body surface stretching device in accordance with the present invention;

Figures 10 and 11 illustrate operation of a third embodiment of a body surface stretching device in accordance with the present invention;

Figure 12 is a side cross-sectional view of a fourth embodiment of a body surface stretching device in accordance with the present invention;

Figure 13 is a side cross-sectional view of a fifth embodiment of a body surface stretching device in accordance with the present invention;

Figure 14 is an exploded perspective view of an alternate embodiment of a microprotrusion array which can be used with the present invention; and

Figure 15 is a graph of tension or force applied versus skin strain.

**MODES FOR CARRYING OUT THE INVENTION**

Each of the embodiments illustrated in the Figures for the present invention stretch the body surface or skin 30 after pathways 31 have been formed by piercing the body surface with a plurality of microprotrusions 34. The stretching or tensioning of the skin maintains the pathways open and preferably enlarges the cross sectional area of the pathways 31 in the skin. Thus, small cuts, slits, or holes called pathways are made in the outermost layer of the body surface to a limited depth by treating the body surface with a device having microprotrusions. Following microprotrusion treatment, the treated body surface is subjected to a predetermined amount of tension (i.e., stress). The skin is stretched in one or more directions by a skin stretching device. The skin stretching/tensioning devices according to the present invention apply a predetermined amount of tension (i.e., stress) in the range of from about 0.01 M Pa to about 10 M Pa (M Pa = megapascal = $1 \times 10^6$ pascals). The preferred range of tension/stress is about 0.05 M Pa to about 2 M Pa. The amount of skin strain resulting from a given tension varies between individuals depending upon skin characteristics, such as the age of the patient, the location of the application on the patient's body, and the tensioning direction. Therefore, in order to adapt to individual characteristics, the skin tensioning devices according to the present invention preferably are designed to provide a given tension (stress) rather than a given strain. In general, for these stress or tension ranges, the applied skin strain is within about 5 to 60% and most preferably within about 10 to 50%. Strain is the amount of skin stretch per unit length of skin and is defined as the change in length of skin caused by stretching divided by the length of the skin in a non-stretched state. The strain of skin can be expressed mathematically by the following equation:

$$\text{Strain} = (I_{ext} - I_{non\text{-}ext}) \div I_{non\text{-}ext}$$

wherein:

$I_{ext}$ is the length of a sample of skin in a stretched state; and
$I_{non\text{-}ext}$ is the length of the skin sample in a non-stretched state.

Stretching the skin keeps the pathways open for longer periods, i.e., pathway closure is postponed. An agent delivery device is placed in contact with the treated and stretched region of the body surface to deliver an agent therethrough.

The present invention improves the agent flux by stretching the outermost layer(s) of the body surface after the pathways have been made. For elongated pathways in the shape of slits, the direction of stretching is preferably transverse to the length of the slits so as to enlarge the cross-sectional area of the slits in the body surface. For circular pathways, the direction of stretching is preferably multi-directional to enlarge the cross-sectional area of the circular holes. For other shaped pathways the direction of stretching may be bidirectional or multidirectional as necessary in order to enlarge the cross-sectional area of the pathways. With each of the embodiments of the present invention, the skin is stretched either after puncturing or during and after puncturing. After penetration by the plurality of microprotrusions 34, the skin is maintained under tension created by stretching the skin to maintain the pathways open through an administration period.

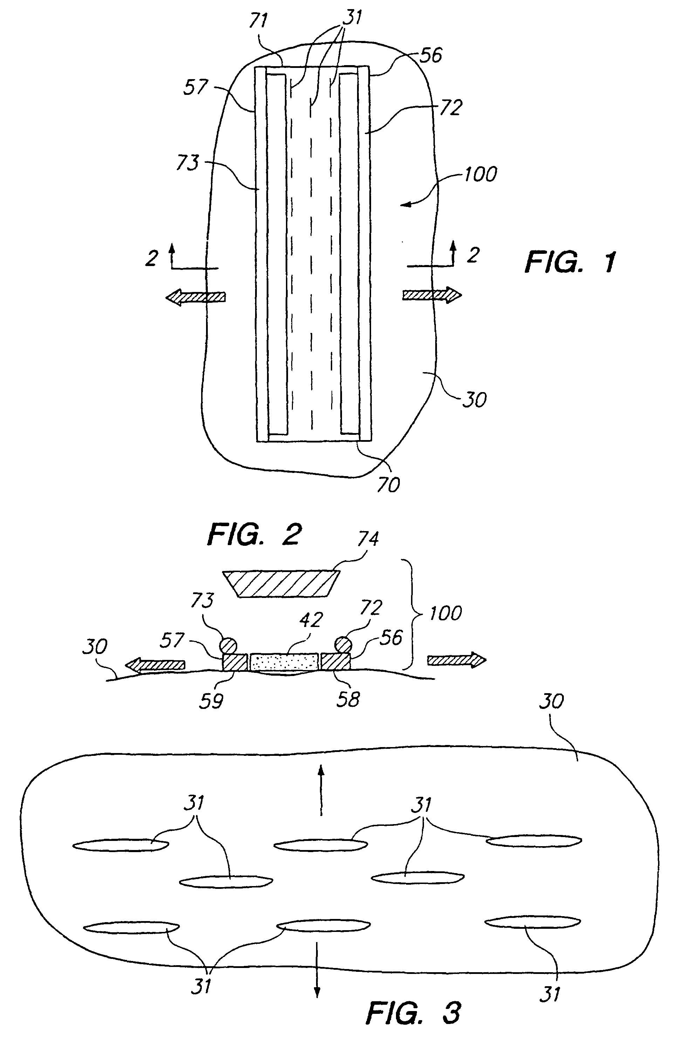

The devices of the present invention, shown in Figures 1, 2, and 4 - 13, provide a tension (i.e., stress) of about 0.01 to about 10 M Pa, and preferably from about 0.05 to about 2 M Pa, to the skin following microprotrusion penetration.

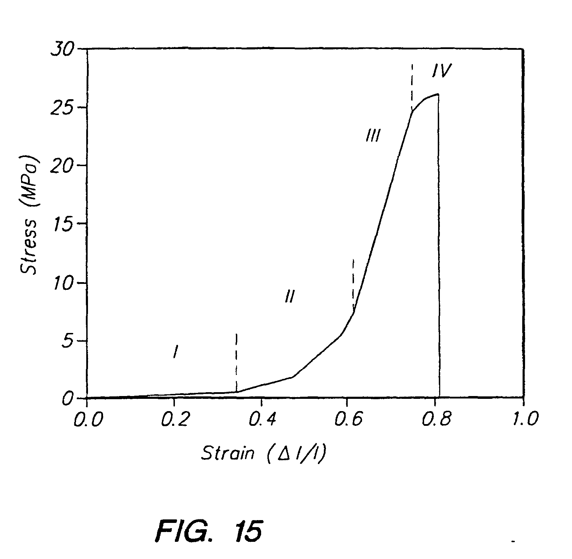

Figure 15 illustrates the typical stress-strain curve for an in vitro tensile test on excised mammalian skin. In phase I there is rapid extension of skin under low load. In phase II there is rapid stiffening of skin followed by phase III in which the skin has stiff behavior. If the skin is tensioned to a degree that reaches phase IV, skin tearing and rupture occurs. For effective skin stretching it is desirable to tension to a degree that results in phase II or III strain but not phase IV strain. Figure 15 illustrates the typical stress-strain curve for unpierced skin, however, the curve may vary somewhat for skin which has been punctured by an array of microprotrusions.

The apparatus of the present invention is for use in the percutaneous administration of an agent. The terms "substance", "agent", and "drug" can be used interchangeably and broadly include physiologically or pharmacologically active substances for producing a localized or systemic effect or effects in mammals, including humans and primates, avians, valuable domestic household, sport or farm animals, or for administering to laboratory animals such as mice, rats, guinea pigs, and the like.

The major barrier properties of the skin, such as resistance to agent permeation, reside with the outermost layer of the skin, i.e., stratum comeum. The inner division, i.e., the backing layers, of the epidermis generally comprises three layers commonly identified as stratum granulosum, stratum malpighii, and stratum germinativum. There is essentially little or no resistance to transport or to absorption of an agent through these layers. Therefore, for enhancing transdermal flux the microprotrusions used to create pathways in the body surface need only penetrate through the stratum comeum in order for the agent to be transdermally delivered with little or no resistance through the skin.

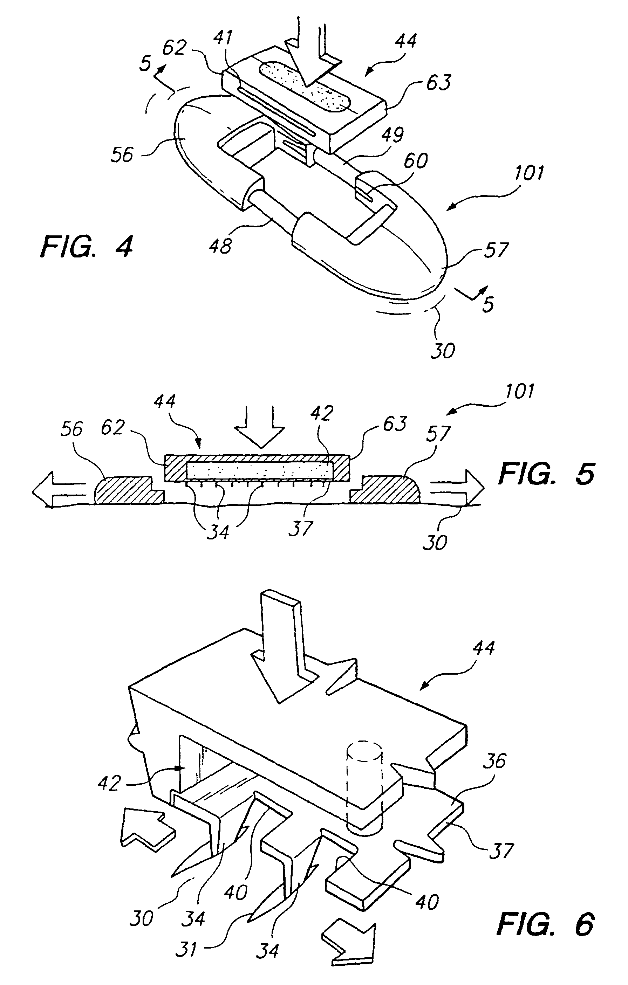

The embodiments of the invention utilize a plurality of microprotrusions to pierce the body surface. Preferably, the microprotrusions are in the form of a microprotrusion array as shown in Figure 6 with a plurality of blade type microprotrusions 34 extending outwardly from one surface of a thin, compliant member or sheet 36. The sheet 36 of the Figure 6 embodiment is oriented during use with a main surface parallel to the patient's body surface 30. The microprotrusions 34 extend from a skin proximal edge of the sheet 36 and are oriented with the planar surfaces of the microprotrusing blades perpendicular to the patient's body surface.

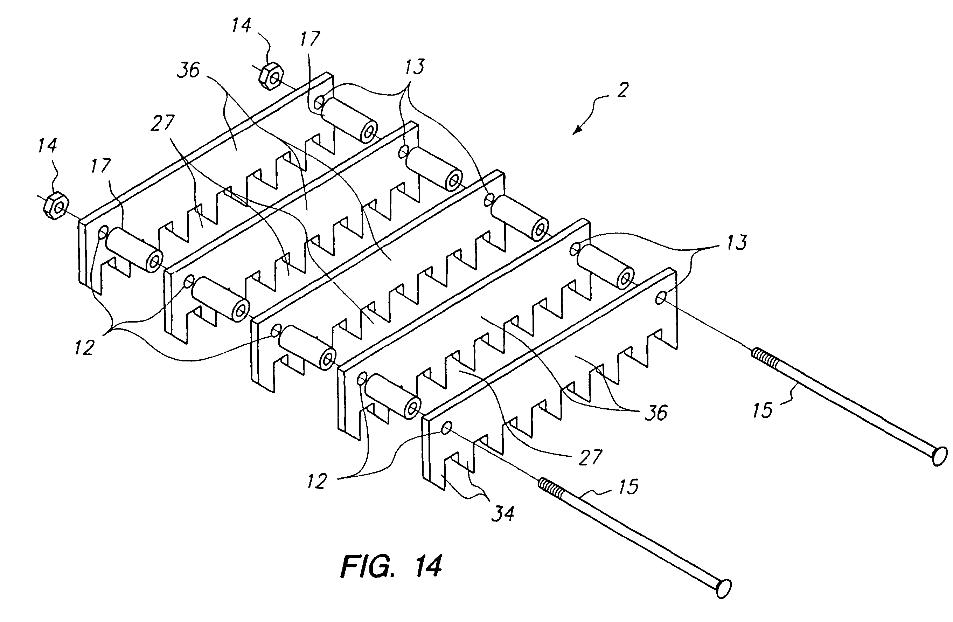

A particularly preferred configuration for the device is illustrated in Figure 14 and comprises a plurality of individual sheet members 36 stacked together to form a microprotrusion device 2. Each thin sheet 36 in use is oriented perpendicular to the patient's body surface 30. The sheets 36 each have a plurality of microprotrusions 34 in the same plane as the sheet 36 which extend outward from a body proximal edge 38 of the sheet 36 for penetrating the body surface 30. Each of the sheet members 36 has a pair of holes 12, 13 through which bolts 15 are inserted. Spacers (e. g., tubes) 17 are positioned between each adjacent pair of sheet members 36 to form voids 27 therebetween. The spaced sheet members 36 are held together as a unit by passing the bolts 15 through the sheet members and spacers 17 and securing nuts 14 on the ends of the bolts, or using other known fasteners. The voids 27 can be filled with a reservoir matrix material (e.g., a gel) adapted to contain the beneficial agent to be delivered. Those skilled in the art will appreciate that spacers 17 having other than tube-like configurations (e.g., square or rectangular blocks) can also be used to provide voids 27 between the agent reservoir 42 (i.e., the agent reservoir contained in the voids 27) and the skin. Furthermore, more than two sets of bolts 15, or other fastening pins, may be used to secure the sheet members 36 and spacers 17 together.

Although either of these two configurations are preferred because of the many advantages they afford over the prior art, the present invention can be used with other known microprotrusion arrays, for example, those described in U.S. Patent Nos. 5,279,544 issued to Gross et al.; 3,964,482 issued to Gerstel et al.; 5,250,023 issued to Lee et al.; Reissue 25,637; 5,312,456 issued to Reed et al.; and disclosed in PCT Publication Nos. WO97/48440; WO96/37256; WO97/03718; WO98/11937; and WO98/00193. The sheet member shown in Figure 14 may alternatively be coiled in a loose spiral or folded in a serpentine configuration. Alternatively, a plurality of cylindrical sheet members may be placed in concentric circles.

Either of the configurations of microprotrusion arrays shown in Figures 6 and 14 can be embodied in separate devices that are used to pretreat the patient's body surface and then removed so that the skin stretching and drug reservoir device of the present invention can be applied to tne pretreated area. Other pretreatment skin perforating devices can be used in conjunction with the present invention. For example, the device described in U.S. Patent No. 5,611,806 issued to Jang comprises a plurality of alternately disposed needle disks and spacers which are combined together for rotational movement as a unit. The device is rolled over the skin to form a plurality of fissures.

Figures 1 and 2 illustrate a device 100 for stretching a body surface 30 having a plurality of preformed pathways 31 in the body surface. The pathways 31 may be formed with the microprotrusions 34 as shown in Figures 6 or 14 or with a rolling tool, for example as described in U.S. Patent No. 5,611,806 issued to Jang. The device 100 has an agent delivery reservoir 42 located between a pair of elongated opposing skin stretching members 56 and 57 held together by expandable elements 70 and 71 (see Figure 1 in which reservoir 42 is removed to show the fissures 31). The elongated members 56 and 57, as clearly shown in Figure 1, are arranged so that the axes of members 56 and 57 are roughly parallel with the lengths (i.e., the longer dimension) of the slits 31. The elongated opposing skin stretching members 56 and 57 have skin engaging surfaces 58 and 59, e.g., adhesive-coated surfaces 58 and 59, which engage and adhere to the body surface 30. A pair of cylindrical elements 72 and 73 are attached to the skin distal sides of the members 56 and 57, respectively. A wedge 74 is pressed vertically, i.e., toward the skin, between the cylindrical elements 72 and 73 which displaces the members 56 and 57 in the direction of the arrows. This displacement of members 56 and 57 creates transverse tension in the body surface 30 between the members 56 and 57 and enlarges the cross-sectional area of the pathways 31 as illustrated in Figure 3 to provide increased open area for agent transport. A locking mechanism (not shown in the Figures) can be provided within device 100 to lock the wedge into place between cylindrical elements 72 and 73 in their displaced (i.e., skin stretched) position. The agent is transported from the reservoir 42 through the open pathways 31. The tension can be relieved and reapplied several times to reopen the pathways 31, if necessary, during transdermal agent delivery. Likewise, the wedge 74 can be inserted farther at a subsequent time to increase the skin tension progressively over time.

As best illustrated in Figure 1, for a given area of skin 30 to be stretched, the expandable skin stretching members 56 and 57 are preferably in an elongated configuration, i.e., they preferably have a length much greater than the distance separating members 56 and 57. In other words, the distance separating the skin stretching members 56 and 57 is small compared to the length of the members 56 and 57. In this manner, a relatively large area of skin 30 can be stretched by relatively small lateral movement of the members 56 and 57 in the direction of the arrows shown in Figures 1 and 2. This configuration is likewise preferred from the standpoint of adhering members 56 and 57 to the surface of skin 30 since the adhesive forces are highest per unit area in this configuration.

In the embodiment shown in Figures 4 and 5, a stretching device 101 uses biasing members such as springs (not shown in Figures 4 and 5) housed in cylindrical members 48 and 49 to stretch the body surface 30 after pathways have been created with the microprotrusions 34 on the cartridge 44. The expandable device 101 has an adhesive coating on the skin contacting surfaces of opposing skin stretching members 56 and 57. The adhesive coating is initially protected by a release liner (not shown) which is removed just prior to placing the device 101 on the skin. The opposite ends 56 and 57 of the device 101 are initially held together manually or mechanically with a retainer such that the biasing members located within the cylindrical members 48 and 49 are in compression. The patient removes the release liner and applies the device 101 to a portion of the skin surface 30 such that the adhesive on the underside holds the device 101 to the patient's skin surface 30. A snap-in cartridge 44 having an array of microprotrusions 34 on a skin contacting surface and a reservoir 42 therein is then snapped into the opening between the two opposing ends 56 and 57 such that protrusions 41 on each side (only one shown in Figure 4) of the housing 44 lock into the indentations 60 (only one shown) in the device 101 as the microprotrusions 34 pierce the outermost layer of the skin surface 30. Then the patient removes a disposable retainer (not shown) from the device 101 to release the biasing members inside the cylindrical members 48 and 49 which are in compression so that the device 101 expands to stretch the skin.

In an optional embodiment not illustrated, the ends 62 and 63 of the cartridge 44 form a wedge to match inclined surfaces in the device 101 so that when the cartridge 44 is snapped into the expandable device 101, the ends 62 and 63 maintain the skin in a stretched condition after penetration of the body surface by the microprotrusions 34. As seen in Figure 6, the stretching of the body surface 30 in a direction transverse to the length (i.e., the longer dimension) of the slits 31 (and also transverse to the plane of the microprotrusions 34) holds the slits 31 open during agent delivery.

Figures 5 and 6 have been illustrated with a snap in cartridge 44 containing both the microprotrusions 34 and the reservoir 42. When the cartridge 44 having the microprotrusions 34 and reservoir 42 is used, the skin is preferably stretched prior to application of the microprotrusions. Alternatively, the skin may be pretreated to form pathways 31 prior to stretching and application of an agent delivery cartridge. The stretching of the skin 30 is maintained through an agent (e.g., drug) administration period.

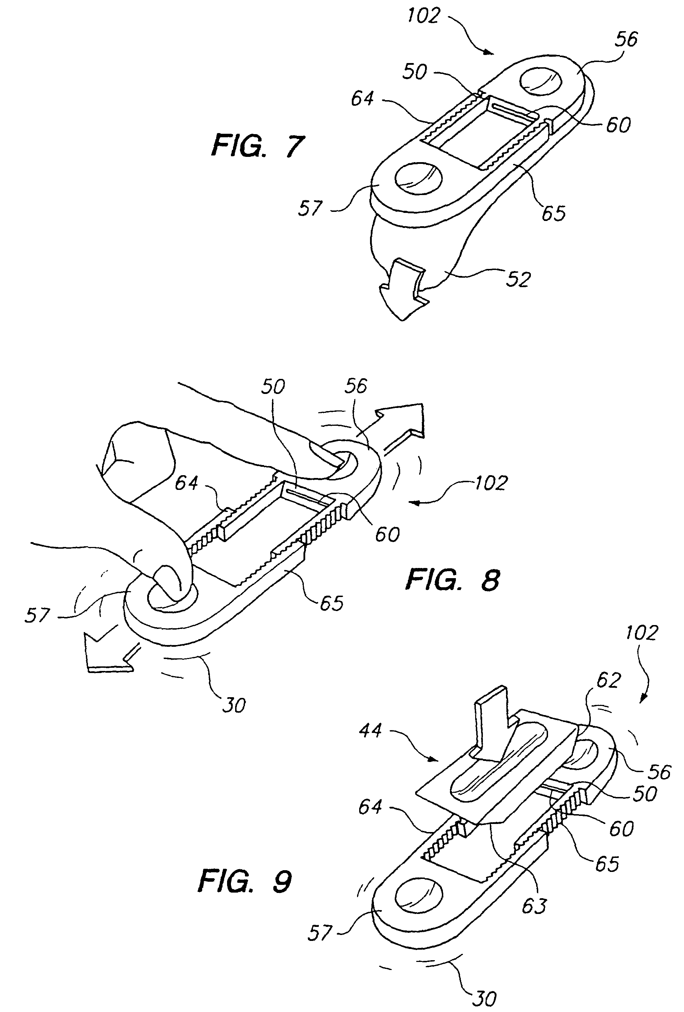

In the embodiment illustrated in Figures 7 through 9, an expandable device 102 is manually actuated by the patient, but essentially retains the same characteristics of operation as described with respect to the earlier embodiments, in that the skin is stretched after puncturing. With respect to this embodiment, pathways are made in the body surface using one of the pretreatment devices described previously. Then the release liner 52 is removed to expose the adhesive on the underside of each of the ends 56 and 57 of the device 102, and the device is placed on the patient's pretreated body surface. The patient or another person then stretches the body surface 30 by spreading apart the ends 56 and 57 of the device 102 (as shown in Figure 8) a predetermined amount which achieves a skin tension in the range of about 0.01 to about 10 M Pa, and preferably about 0.05 to about 2 M Pa. The ratcheted sides 64 and 65 on the expandable device 102 allow the device to maintain its expanded position after the patient or another medical technician has removed their hand from the device. The snap-in cartridge 44 having no microprotrusions is then pressed down with a load applied normal to the body surface as described with respect to the earlier embodiment. If the snap-in cartridge housing 44 has the optional wedge shape, then the insertion of the housing 44 into device 102 can be used to achieve the predetermined level of skin stretching. The snap in cartridge housing 44 has a drug-containing reservoir which provides transdermal drug delivery. It is within the scope of the present invention for the cartridge 44 to have microprotrusions 34 on the body surface thereof to create additional pathways.

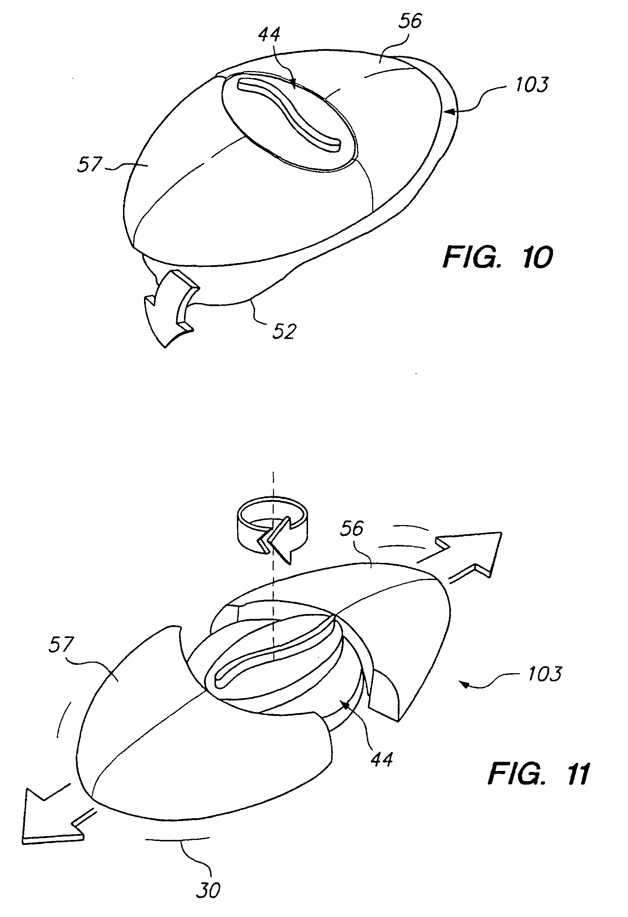

According to a further alternative embodiment a stretching device 103 illustrated in Figures 10 and 11 is operated with a rotational motion, rather than a translational motion, in order to move the opposing ends 56 and 57 a predetermined distance apart which achieves a skin tension in the range of about 0.01 to about 10 M Pa, and preferably about 0.05 to about 2 M Pa. The device 103 is an integral unit, rather than having a separate cartridge 44 with the agent reservoir therein, as in devices 100-102. The patient or another person pretreats the body surface 30 to create the pathways therein then removes the release liner 52 from the underneath side of the device 103 to expose the adhesive on the skin proximal surface of each end 56 and 57, and places the device 103 on the patient's body surface 30. The reservoir housing 44 is then rotated to bring the agent reservoir (not shown) into contact with the body surface 30, and due to its elliptical shape, forces the ends 56 and 57 of the expandable device 103 apart to stretch the body surface 30 transverse to the length of the pathways created by the pretreatment.

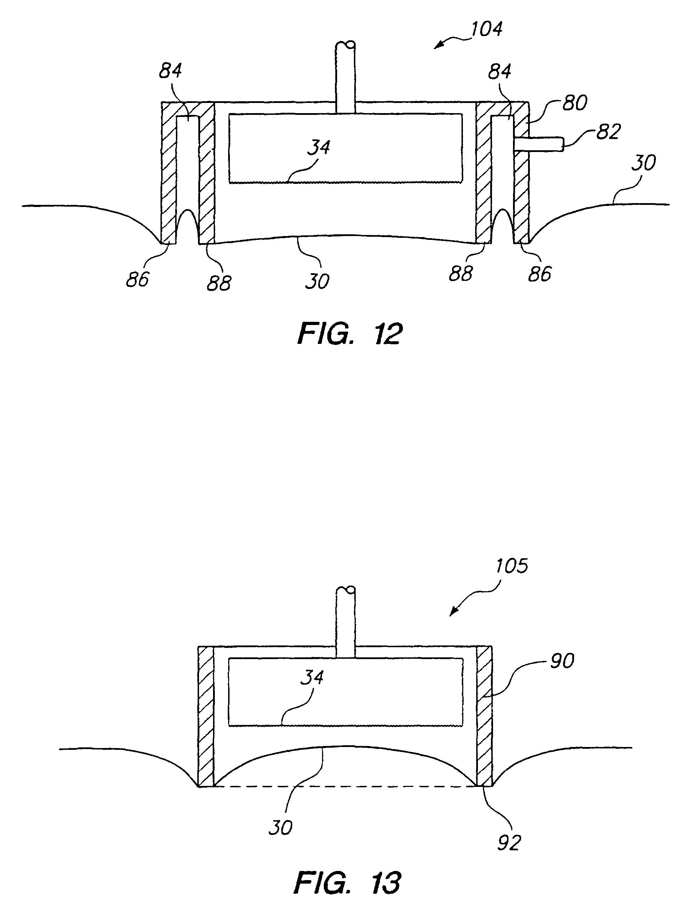

A fifth embodiment of the invention is illustrated in Figure 12. In this embodiment, the device 104 includes a suction member 80 of a rectangular, square, circular, or other shape in plan view connected to a tube 82 for drawing a suction. The suction member 80 has a suction channel 84. For multi-directional stretching, the suction channel 84 may be a continuous or substantially continuous channel having a rectangular, circular, oval, or other shape in plan view. For uni-directional stretching, two opposed suction channels 84 may be provided. An outer edge of the suction member 80 includes a lower surface 86 which grips the skin by having a high friction coefficient with respect to the skin. A lower surface 88 of an inner edge of the channel 84 is preferably provided with a low friction surface which allows the skin to slide over the inner surface. The non-slip surface 86 may be an adhesive, while the slip surface 88 may be coated with a lubricant. When a suction is applied to the suction tube 82 a low pressure area is provided within the channel 84 which draws the skin 30 into the channel as shown in Figure 12, stretching the skin within the opposing surfaces 88. The amount of suction applied within channel 84 will vary depending on the size of device 104. Those persons of ordinary skill in the art can determine the level of suction needed to achieve a skin tension in the range of about 0.01 to about 10 M Pa, and preferably about 0.05 to about 2 M Pa. The skin is treated with an array of microprotrusions 34 either before or after stretching. A cartridge (not shown) is inserted into an interior of the device 104 and placed against the stretched skin for agent delivery.

A sixth embodiment of the invention is illustrated in Figure 13. The device 105 includes a tubular member 90 having a lower edge 92 which is pressed into the skin surface 30 causing skin in a center of the tubular member to form a dome shape and become tensioned. The amount of skin stretching or tensioning may be controlled by the amount of pressure applied to the tubular member 90. Manually applied pressure is not recommended in this embodiment since the amount of skin tension achieved through manually applied downward pressure applied to member 90 will be variable and difficult to ensure a skin tension in the range of about 0.01 to about 10 M Pa. Thus with this embodiment, a device (not shown) for applying a predetermined downward (i.e., toward the skin) force to the tubular member 90 is recommended. To prevent slippage between the skin and the lower edge 92 of the tubular member 90, a non-slip surface, such as an adhesive may be employed. The array of microprotrusions 34 are then applied before or after tensioning of the skin. A cartridge (not shown) is inserted in a center of the tubular member 90 and placed against the treated skin with enlarged pathways to deliver an agent. The device 105 as shown in Figure 13 may be cylindrical, square, rectangular, or any other shape in plan view.

A seventh embodiment of the invention which is not illustrated in the figures comprises an elastic strap which is adapted to extend partially, but not completely, around a limb (i.e., an arm, leg or finger) of a patient. The elastic strap has adhesive coated at the ends thereof which adhesive is adapted to be placed in contact with the skin on the limb. A skin site on the limb to be stretched is first identified. The strap is applied by first removing an adhesive liner at one end of the strap and adhering that end of the strap adjacent to the skin site to be stretched. The elastic strap is then extended around the limb, in a condition having a predetermined amount of stretch (i.e., sufficient stretch to achieve a skin tension in the range of about 0.01 to about 10 M Pa, and preferably about 0.05 to about 2 M Pa) and the other end of the strap is adhered adjacent the opposite side of skin site to be stretched. The stretching of the elastic strap stretches the skin site positioned between the two adhered end portions of the strap.

The expandable devices 100 through 105 of any of the embodiments can be held in contact with the body surface 30 in any of a variety of ways, such as but not limited to adhesive, tape, a strap, or an elastic bandage.

In the preferred embodiments, the microprotrusions 34 are microblades as shown in Figures 6 and 14. In the embodiment shown in Figure 6, the sheet member 36 is formed with a plurality of openings 40 adjacent the microprotrusions 34 to permit the transport of agent from an agent reservoir 42 located on the skin distal side of sheet 36 within cartridge 44. In this embodiment, the openings 40 correspond to the portion of the sheet member 36 occupied by each of the microprotrusions 34 prior to the microprotrusions being bent into a position which is substantially perpendicular to the plane of the sheet member 36 as shown.

The preferred configurations for the array of microprotrusions and a connecting medium for delivering agents from the reservoir 42 to the body surface are described in detail in WO 97/48440; WO 97/48441; WO 97/48442; and WO 98/28037.

The array of microprotrusions 34 in the various embodiments of the present invention may take on different shapes. The present invention can be used with any known delivery device and is not limited to any particular device. It will be appreciated by those working in the field that the present invention can be used in conjunction with a wide variety of electrotransport systems, as the invention is not limited in any way in this regard. For example, the apparatus of the present invention can be used with the electrotransport systems disclosed in U.S. Patent Nos. 5,147,296 issued to Theeuwes et al., 5,080,646 issued to Theeuwes et al., 5,169,382 issued to Theeuwes et al., 5,423,739 issued to Phipps et al., 5,385,543 issued to Haak et al., 5,310,404 issued to Gyory et al., and 5,169,383 issued to Gyory et al., and PCT Publication No. WO 97/48440. Similarly, any known passive transdermal delivery device can be used with the present invention, as the invention is not limited in this regard. For example, the apparatus of the present invention can be used with the passive systems disclosed in U.S. Patent Nos. 4,379,454 issued to Campbell et al., 4,588,580 issued to Gale et al., 4,832,953 issued to Campbell et al., 4,698,062 issued to Gale et al., 4,867,982 issued to Campbell et al., and 5,268,209 issued to Hunt et al., and PCT Publication No. WO 97/48440. It will be appreciated by those working in the field that the present invention can also be used in conjunction with a wide variety of osmotic and pressure driven systems, as the invention is not limited to a particular device in this regard. For example, the apparatus of the present invention can be used with the osmotic and pressure driven systems disclosed in U.S. Patent Nos. 4,340,480 issued to Pall et al., 4,655,766 issued to Theeuwes et al., 4,753,651 issued to Eckenhoff et al., 5,279,544 issued to Gross et al., and 5,242,406 issued to Gross et al.

The following example illustrates the utility of the present invention in enhancing transdermal polypeptide flux.

**EXAMPLE**

In this experiment, a radio-labelled synthetic model decapeptide was delivered by passive diffusion through the skin of live hairless guinea pigs at skin sites that were pretreated by piercing with an array of microprojections. The purpose of the experiment was to determine whether stretching the skin following the microprojection pretreatment improved flux of the drug through the skin in vivo. The decapeptide had a molecular weight of 1141 daltons and a water solubility of greater than 50 mM over a wide pH range. The decapeptide had been previously found to exhibit insignificant (i.e., less than 0.1 $\mu$g/cm$^2$hr) passive diffusional flux through untreated skin.

Twelve hairless guinea pigs were divided into two groups of six. The first group (test group) had skin under tension during transdermal decapeptide delivery. The second group (control group) had skin under normal, relaxed (i. e., no tension) conditions during transdermal decapeptide delivery. Both groups had skin sites under tension when the microprotrusion arrays were applied since previous studies have shown that stretching the skin at the time of microprotrusion application helps the microprotrusions to more consistently pierce the skin. The microprotrusion arrays used in the study had the configuration of a metal sheet 36, with a multiplicity of openings 40 and microprotrusions 34 as shown in Figure 6. Each array was a stainless steel sheet having a thickness of 0.025 mm (1 mil) having a multiplicity of photoetched and punched microprotrusions extending roughly perpendicular to the plane of the sheet. The sheet had an area of 2 cm$^2$, 72 openings and 144 microprojections. The microprojections had a triangular shape with a length 545 $\mu$m. In both groups (test and control), the skin of one flank was stretched manually in two directions ($\swarrow$, and $\updownarrow$) when the microprojection arrays were applied. The bi-directional stretching was estimated to achieve a skin tension of between 0.1 and 1 M Pa. The microprotrusion arrays were positioned on the skin by first applying a foam double-sided adhesive ring (diameter 3.8 cm, thickness 0.16 cm with a 2 cm$^2$ central opening) on the skin site. Next, the microprotrusion array was layed in the opening, microprotrusion side down. Next, the skin distal side of the microprotrusion array was struck using a spring-loaded applicator. Following microprotrusion piercing, the manually applied stretching tension was released.

In the test group, the adhesive ring was left in place and served to keep the skin under the drug compartment (i.e., the drug formulation-filled opening in the foam ring) in the stretched configuration. After the microprojection array was removed, a hydrogel containing an aqueous solution (pH 6) of the tritiated decapeptide in an hydroxy ethyl cellulose gel 10 mM, 2% HEC) was dispensed into the opening in the foam ring and a plastic cover was applied to the adhesive outer surface of the ring to seal the drug formulation.

In the control group, the microprojection array and the double-sided adhesive foam ring were removed following microprotrusion piercing causing the stretching tension to be released and the skin to return to its normal relaxed (i.e., unstretched) state. An adhesive ring was placed over the skin site in a relaxed state and the same formulation was dispensed into the opening in the foam ring, which was subsequently sealed with a plastic cover.

At 1 and 24 h after applying the decapeptide formulation to the animals' skin, three animals from each group were removed and residual drug washed from the skin. The amount of drug penetrated during these time intervals was determined by measuring urinary excretion of tritium (previous studies had shown that in hairless guinea pigs, 65% of the tritium derived from tritiated decapeptide injected intravenously is excreted in urine). The results (Table 1) show that keeping the microslit skin in a stretched state during passive transdermal decapeptide delivery increases the amount of decapeptide delivered by several fold for up to 24 h.

Table 1

| Animal Group | Mean Decapeptide Delivered ($\mu$g) | | Mean Decapeptide Flux Per Unit Area ($\mu$g/cm$^2$h) | |
|---|---|---|---|---|
| | Application Time (h) | | Application Time (h) | |
| | 1 h | 24 h | 1 h | 24 h |
| Test (stretching) | $27.9 \pm 3.3$ | $94.7 \pm 23.7$ | $13.9 \pm 1.6$ | $1.97 \pm 0.49$ |
| Control (no stretching) | $9.8 \pm 0.8$ | $23.5 \pm 5.9$ | $4.9 \pm 0.4$ | $0.49 \pm 0.12$ |

The mean decapeptide flux per unit area was also calculated at the mid-time points of the 1 and 24 hour periods (i.e., at 0.5 hours and 11.5 hours). The results (Table 2) show that the decrease in flux per unit area is less with stretching of the skin compared to no stretching of the skin. These results suggest that stretching of the skin following microprojection piercing and during transdermal drug delivery retards the closure of the microslits in the skin.

**EP 1 239 917 B1**

Table 2

| Animal Group | Mean Decapeptide Flux per Unit Area ($\mu$g/cm$^2$ h) | |
|---|---|---|
| | Mid-time (h) | |
| | 0.5 h | 12.5 h |
| Test (stretching) | 13.9 $\pm$ 1.6 | 1.45 $\pm$ 0.49 |
| Control (no stretching) | 4.9 $\pm$ 0.4 | 0.30 $\pm$ 0.12 |

**Claims**

1. Apparatus for delivering an agent through a body surface (30) having a plurality of micropathways (31) therein, the apparatus comprising:

   an agent reservoir (42) for containing the agent to be delivered and placement in agent-transmitting relation with the body surface (30) and the micropathways (31);
   a stretching device having body surface engaging portions (56, 57, 86, 88, 92) for at least partially surrounding the body surface site (30) to be stretched; and
   a body surface stretching mechanism (72, 73, 74, 82, 84) associated with the body surface engaging portions (56, 57, 86, 88, 92) for applying a tension to the at least partially surrounded body surface site during agent delivery therethrough;

   **characterised in that** the body surface stretching mechanism (72, 73, 74, 82, 84) can applying a tension of 0.01 to 10 M Pa to the at least partially surrounded body surface site during agent delivery therethrough, and the stretching device comprises opposing first and second body surface engaging portions (56, 57), wherein the reservoir (42) is positionable between the first and second portions (56, 57).

2. Apparatus according to claim 1, wherein the first and second portions (56, 57) have adhesive surfaces (58, 59) which contact and adhere to the body surface (30).

3. Apparatus according to claim 1 or 2, wherein the first and second portions (56, 57) are moveable away from each other.

4. Apparatus according to any preceding claim, comprising a biassing member (48, 49) for biassing the first and second portions (56, 57) apart.

5. Apparatus according to claim 4, further comprising a retainer which initially holds the first and second portions (56, 57) in a first position, and upon removal of the retainer the biassing member (48, 49) biasses the first and second portions (56, 57) apart.

6. Apparatus according to claim 3, wherein the reservoir (42) comprises a wedge-shaped housing (62) which contacts an inclined surface (50) on the first and/or the second portion (56, 57) to move the first and second portions (56, 57) apart when the housing (62) is inserted therebetween.

7. Apparatus according to claim 6, further comprising a holding member (64, 65) for holding the stretching device in an expanded state after the first and second portions (56, 57) have been moved apart.

8. Apparatus according to any one of claims 3 to 7, wherein the reservoir (42) is in a rotatable housing (44) which is rotatable to move the first and second portions (56, 57) apart.

9. Apparatus according claim 3, wherein the first and second portions (56, 57) are connected by an expandable member (64, 65), and which further comprises a spreader (44) inserted between the first and second portions (56, 57).

10. Apparatus according to any preceding claim, wherein a body surface-contacting face (37) of the reservoir (42) has a plurality of microprotrusions (34) which can penetrate the body surface (30) to form the micropathways (31).

11. Apparatus according to any preceding claim, wherein the reservoir is located in a housing (62) having a plurality of microprotrusions (34) extending from a body surface-contacting face (37) of the housing (62) which can penetrate the body surface (30) to form the micropathways (31).

12. Apparatus according to claim 10 or 11, which in use also stretches the body surface (30) during microprotrusion penetration.

13. Apparatus according to claim 12, wherein in use the body surface engaging portions (56, 57) stretch the body surface (30) in a direction transverse to the largest dimension of the micropathways (31).

14. Apparatus according to any one of claims 10 to 13, wherein the microprotusions (34) are for forming elongate microslits.

15. Apparatus according to any one of claims 10 to 14 wherein the microprotusions (34) are for forming micropathways (31) in the stratum corneum of skin.

16. Apparatus according to claim 1, wherein the stretching device includes a device (82, 84) for applying suction to the body surface (30).

17. Apparatus according to claim 16, wherein in use the apparatus stretches the body surface (30) in multiple directions.

18. Apparatus according to any preceding claim, wherein the stretching mechanism (72, 73, 74, 82, 84) applies a tension of 0.05 to 2 M Pa to the at least partially surrounded body surface site during agent delivery therethrough.

**Patentansprüche**

1. Vorrichtung zur Abgabe eines Wirkstoffs durch eine Körperoberfläche (30) mit einer Vielzahl von Mikrodurchgängen (31), wobei die Vorrichtung umfasst:

   ein Wirkstoffreservoir (42) zur Aufnahme des abzugebenden Wirkstoffs und zum Anbringen in einer den Wirkstoff übertragenden Anordnung mit der Körperoberfläche (30) und den Mikrodurchgängen (31);
   eine Dehnvorrichtung mit Teilen (56, 57, 86, 88, 92), die in die Körperoberfläche greifen, um die zu dehnende Stelle der Körperoberfläche (30) mindestens teilweise zu umgeben; und
   einen Körperoberflächen-Dehnmechanismus (72, 73, 74, 82, 84), der zu den Teilen (56, 57, 86, 88, 92), die in die Körperoberfläche greifen, gehört, um eine Spannung auf die mindestens teilweise umgebene Körperoberfläche auszuüben, während der Wirkstoff durch sie hindurch abgegeben wird;

   **dadurch gekennzeichnet, dass** der Körperoberflächen-Dehnmechanismus (72, 73, 74, 82, 84) eine Spannung von 0,01 bis 10 M Pa auf die mindestens teilweise umgebene Stelle der Körperoberfläche ausübt,: während der Wirkstoff durch sie hindurch abgegeben wird, und dass die Dehnvorrichtung gegenüberliegende erste und zweite Teile (56, 57), die in die Körperoberfläche greifen, umfasst, wobei das Reservoir (42) zwischen den ersten und zweiten Teilen (56, 57) angeordnet werden kann.

2. Vorrichtung nach Anspruch 1, worin die ersten und zweiten Teile (56, 57) Klebeflächen (58, 59) aufweisen, die die Körperoberfläche (30) berühren und daran kleben.

3. Vorrichtung nach den Ansprüchen 1 oder 2, worin die ersten und zweiten Teile (56, 57) voneinander weg bewegt werden können.

4. Vorrichtung nach einem der vorhergehenden Ansprüche, umfassend ein Spannelement (48, 49), um die ersten und zweiten Teile (56, 57) auseinander zu spannen.

5. Vorrichtung nach Anspruch 4, die weiterhin einen Halter :umfasst, der anfängtich die ersten und zweiten Teile (56, 57) in einer ersten Position hält, und wobei beim Herausnehmen des Halters das Spannelement (48, 49) die ersten und zweiten Teile (56, 57) auseinander spannt.

6. Vorrichtung nach Anspruch 3, worin das Reservoir (42) ein keilförmiges Gehäuse (62) umfasst, welches eine geneigte Fläche (50) am ersten und/oder zweiten Teil (56, 57) berührt, um die ersten und zweiten Teile (56, 57) auseinander zu schieben, wenn das Gehäuse (62) zwischen ihnen eingesetzt wird.

**7.** Vorrichtung nach Anspruch 6, die weiterhin ein Haltelement (64, 65) umfasst, um die Dehnvorrichtung im expandierten Zustand zu halten, nachdem die ersten und zweiten Teile (56, 57) auseinander geschoben wurden.

**8.** Vorrichtung nach einem der Ansprüche 3 bis 7, worin sich das Reservoir (42) in einem drehbaren Gehäuse (44) befindet, welches drehbar ist, um die ersten und zweiten Teile (56, 57) auseinander zu schieben.

**9.** Vorrichtung nach Anspruch 3, worin die ersten und zweiten Teile (56, 57) mit einem expandierbaren Element (64, 65) verbunden sind, und die weiterhin einen Abstandshalter (44) umfasst, der zwischen den ersten und zweiten Teilen (56, 57) eingefügt ist.

**10.** Vorrichtung nach einem der vorhergehenden Ansprüche, worin eine Seite (37) des Reservoirs (42), die die Körperoberfläche berührt, eine Vielzahl von Mikrovorsprüngen (34) hat,: welche die Körperoberfläche (30) durchdringen können, um die Mikrodurchgänge (31) zu bilden.

**11.** Vorrichtung nach einem der vorhergehenden Ansprüche, worin sich das Reservoir in einem Gehäuse (62) mit einer Vielzahl von Mikrovorsprüngen (34) befindet, die aus einer Fläche (37) des Gehäuses (62) herausragen und die Körperoberfläche (30) durchdringen können, um die Mikrodurchgänge (31) zu bilden.

**12.** Vorrichtung nach Anspruch 10 oder 11, die bei der Anwendung während des Durchdringens der Mikrovorsprünge auch die Körperoberfläche (30) dehnt.

**13.** Vorrichtung nach Anspruch 12, worin bei der Anwendung die Teile (56, 57), die in die Körperoberfläche greifen, die Körperoberfläche (30) in einer Richtung quer zur größten Abmessung der Mikrodurchgänge (31) dehnen.

**14.** Vorrichtung nach einem der Ansprüche 10 bis 13, worin die Mikrovorsprünge (34) zur Bildung von Mikroschlitzen dienen.

**15.** Vorrichtung nach einem der Ansprüche 10 bis 14, worin die Mikrovorsprünge (34) zur Bildung von Mikrodurchgängen (31), im Stratum Comeum der Haut dienen.

**16.** Vorrichtung nach Anspruch 1, worin die Dehnvorrichtung eine Vorrichtung (82, 84) zum Saugen auf der Körperoberfläche (30) umfasst.

**17.** Vorrichtung nach Anspruch 16, worin die Vorrichtung bei der Anwendung die Körperoberfläche (30) in mehreren Richtungen dehnt.

**18.** Vorrichtung nach einem der vorhergehenden Ansprüche, worin der Dehnmechanismus (72, 73, 74, 82, 84) eine Spannung von 0,05 bis 2 M Pa auf die mindestens teilweise umgebene Stelle der Körperoberfläche ausübt, während der Wirkstoff durch sie hindurch abgegeben wird,

## Revendications

**1.** Appareil pour administrer un agent à travers une surface corporelle (30) dans laquelle il y a une pluralité de micro-voies (31), l'appareil comprenant :

- un réservoir d'agent (42) pour contenir l'agent devant être administré et une mise en place en relation de transmission d'agent avec la surface corporelle (30) et les micro-voies (31) ;
- un dispositif d'étirement ayant des parties (56, 57, 86 , 88, 92) d'engagement de la surface corporelle pour au moins partiellement entourer le site de surface corporelle (30) à étirer ; et
- un mécanisme (72, 73,: 74, 82, 84) d'étirement de la surface corporelle, associé avec les parties (56, 57, 86, 88, 92) d'engagement de la surface corporelle pour appliquer une tension au site de surface corporelle au moins partiellement entouré pendant l'administration d'agent à travers celui-ci;

**caractérisé par le fait que** le mécanisme (72, 73, 74, 82, 84) d'étirement de la surface corporelle peut appliquer une tension de 0,01 à 10MPa au site de surface corporelle au moins partiellement entouré pendant l'administration d'agent à travers celui-ci, et le dispositif d'étirement comprend des première et seconde parties opposées (56, 57) d'engagement de surface corporelle, le réservoir (42) étant positionnable entre les première et seconde parties (56, 57).

2. Appareil selon la revendication 1, dans lequel les première et seconde parties: (56, 57) ont des surfaces adhésives (58, 59) qui viennent en contact avec, et adhèrent à, la surface corporelle (30).

3. Appareil selon l'une des revendications 1 ou 2, dans lequel les première et seconde parties (56, 57) sont déplaçables pour être éloignées l'une de l'autre.

4. Appareil selon l'une quelconque des revendications précédentes, comprenant un élément de sollicitation (48, 49) pour solliciter les première et seconde parties (56, 57) pour qu'elles s'écartent l'une de l'autre.

5. Appareil selon la revendication 4, comprenant en outre un organe de retenue qui initialement maintient les première et seconde parties (56, 57) dans une première position, et lors de l'enlèvement de l'organe de retenue l'élément de sollicitation (48, 49) sollicite les première et seconde parties pour qu'elles s'écartent l'une de l'autre.

6. Appareil selon la revendication 3, dans lequel le réservoir (42) comprend un boîtier (62) en forme de coin qui vient en contact avec une surface inclinée (50) sur la première et/ou la seconde partie (56, 57) pour écarter les première et seconde parties (56, 57) lorsque le boîtier (62) est introduit entre elles.

7. Appareil selon la revendication 6, comprenant en outre un élément de maintien (64, 65) pour maintenir le dispositif d'étirement dans un état étendu après que les première et seconde parties (56, 57) ont été écartées l'une de l'autre.

8. Appareil selon l'une quelconque des revendications 3 à 7, dans lequel le réservoir (42) est dans un boîtier rotatif (44) que l'on peut faire tourner pour écarter les première et seconde parties (56, 57).

9. Appareil selon la revendication 3, dans lequel les première et seconde parties (56, 57) sont connectées par un élément extensible (64, 65), et qui comprend en outre un écarteur (44) introduit entre les première et seconde parties (56, 57).

10. Appareil selon l'une quelconque des revendications précédentes, dans lequel une face (37) de contact avec :la surface corporelle du réservoir (42) a plusieurs micro-saillies (34) qui peuvent pénétrer la surface corporelle (30) pour former les micro-voies (31).

11. Appareil selon l'une quelconque des revendications précédentes, dans lequel le réservoir est situé dans un boîtier (62) ayant plusieurs micro-saillies (34) s'étendant à partir d'une face (37) de contact avec la surface corporelle du boîtier (62) qui peut pénétrer la surface corporelle (30) pour former les micro-voies (31).

12. Appareil selon l'une des: revendications 10 ou 11, lequel, lors de l'utilisation, étire également la surface corporelle (30) pendant la pénétration des micro-saillies.

13. Appareil selon la revendication 12, dans lequel, lors de l'utilisation, les parties (56,: 57) d'engagement de la surface corporelle étirent la surface corporelle (30) dans une direction transversale à la dimension la plus grande des micro-voies (31).

14. Appareil selon l'une quelconque des revendications 10 à 13, dans lequel les micro-saillies (34) sont aptes à former des micro-fentes allongées.

15. Appareil selon l'une quelconque des revendications 10 à 14, dans lequel les micro-saillies (34) sont aptes à former des micro-voies (31) dans le stratum corneum de la peau.

16. Appareil selon la revendication 1, dans lequel le dispositif d'étirement comprend un dispositif (82, 84) pour appliquer une succion à la.surface corporelle (30).

17. Appareil selon la revendication 16, dans lequel, lors de l'utilisation, l'appareil étire la surface corporelle (30) dans plusieurs directions.

18. Appareil selon l'une quelconque des revendications précédentes, dans lequel le mécanisme d'étirement (72, 73, 74, 82, 84) applique une tension de 0,05 à 2 MPa au site de surface corporelle au moins partiellement entouré pendant l'administration d'agent à travers celui-ci.

FIG. 1

FIG. 2

FIG. 3

FIG. 4

FIG. 5

FIG. 6

FIG. 7

FIG. 8

FIG. 9

FIG. 10

FIG. 11

**FIG. 12**

**FIG. 13**

FIG. 14

19

FIG. 15